# EUROPEAN PATENT SPECIFICATION

(11) **EP 3 077 350 B1**
(45) Date of publication and mention of the grant of the patent: **14.02.2018**
(21) Application number: 14809134.1
(22) Date of filing: 28.11.2014
(51) Int. Cl.: C07C 2/10, C07C 11/02, C07C 11/107

(54) **ETHYLENE OLIGOMERIZATION WITH MIXED LIGANDS**
ETHYLENOLIGOMERISIERUNG MIT GEMISCHTEN LIGANDEN
OLIGOMÉRISATION D'ÉTHYLÈNE AU MOYEN DE LIGANDS MIXTES

(30) Priority: 05.12.2013 CA 2835683
(43) Date of publication of application: 12.10.2016
(73) Proprietor: Nova Chemicals (International) S.A., 1700 Fribourg (CH)
(72) Inventor: BROWN, Stephen, Calgary, Alberta T2Z 2G7 (CA); CARTER, Charles, Calgary, Alberta T3A 6L7 (CA); ZORICAK, Peter, Calgary, Alberta T3G 2X7 (CA); GAO, Xiaoliang, Calgary, Alberta T3A 2J1 (CA); SEVERIN, Holly, Calgary, Alberta T2G 3V3 (CA)
(74) Representative: Watson, Robert James
(86) International application number: PCT/IB2014/066438
(87) International publication number: WO 2015/083053

(56) References cited:
- US-A1- 2007 232 481
- US-A1- 2012 172 645
- US-B2- 7 994 363

## Description

### TECHNICAL FIELD

This invention relates to the selective oligomerization of ethylene using a chromium catalyst having a P-N-P ligand.

### BACKGROUND ART

Alpha olefins are commercially produced by the oligomerization of ethylene in the presence of a simple alkyl aluminum catalyst (in the so called "chain growth" process) or alternatively, in the presence of an organometallic nickel catalyst (in the so called Shell Higher Olefins, or "SHOP" process). Both of these processes typically produce a crude oligomer product having a broad distribution of alpha olefins with an even number of carbon atoms (i.e. butene-1, hexene-1, octene-1 etc.). The various alpha olefins in the crude oligomer product are then typically separated in a series of distillation columns. Butene-1 is generally the least valuable of these olefins as it is also produced in large quantities as a by-product in various cracking and refining processes. Hexene-1 and octene-1 often command comparatively high prices because these olefins are in high demand as comonomers for linear low density polyethylene (LLDPE).

Technology for the selective trimerization of ethylene to hexene-1 has been recently put into commercial use in response to the demand for hexene-1. The patent literature discloses catalysts which comprise a chromium source and a pyrrolide ligand as being useful for this process - see, for example, United States Patent ("USP") 5,198,563 (Reagen et al., assigned to Phillips Petroleum).

Another family of highly active trimerization catalysts is disclosed by Wass et al. in WO 02/04119 (now United States Patents 7,143,633 and 6,800,702). The catalysts disclosed by Wass et al. are formed from a chromium source and a bridged diphosphine ligand and are described in further detail by Carter et al. (Chem. Comm. 2002, p 858-9). The two phosphorous (P) atoms are preferably bridged by an amine (N) bridge and hence these ligands are typically referred to as "P-N-P" ligands. As described in the Chem. Comm. paper, the most preferred P-N-P ligands are those in which each P atom is bonded to two phenyl groups and each phenyl group is substituted with an ortho-methoxy group. Hexene-1 is produced with high activity and high selectivity by these catalysts.

Similar P-N-P ligands are disclosed by Blann et al. in WO04/056478 and WO 04/056479 (now US 2006/0229480 and US 2006/0173226). However, in comparison to the ligands of Wass et al., the disphosphine/tetraphenyl ligands disclosed by Blann et al. generally do not contain polar substituents in ortho positions. The "tetraphenyl" diphosphine ligands claimed in the '480 application must not have ortho substituents (of any kind) on all four of the phenyl groups and the "tetraphenyl" diphosphine ligands claimed in '226 are characterized by having a polar substituent in a meta or para position. Both of these types of catalysts reduce the amount of hexenes produced and increase the amount of octene (in comparison to the ligands of Wass et al.) and the catalysts are generally referred to as "tetramerization catalysts". However, the alpha selectivity of the C₆ stream that is coproduced by these tetramerization catalysts is poor. In particular, large amounts of cyclic C₆ molecules (such as methyl cyclopentane and methylene cyclopentane) are observed in the hexene co-product that is produced with these catalysts. The "cyclic C₆" molecules have little or no commercial value. In addition, there is a need to separate these molecules from the alpha hexene (also referred to as hexene-1) and this separation requires energy (and may also require another distillation column).

A family of P-N-P oligomerization ligands that enables the production of octene and coproduct hexene having a high alpha selectivity is disclosed in commonly assigned U.S. 7,994,363 (Carter et al.). However, we have observed that the alpha selectivity of the hexene produced with these catalysts can decrease somewhat as process conditions are optimized to promote very high catalyst activity (of greater than 1 x 10⁶ grams of ethylene consumed per gram of Cr per hour). The present invention uses a mixed catalyst system that enables high catalyst activities; good selectivity to octene and good alpha hexene selectivity.

### DISCLOSURE OF INVENTION

The present invention provides:
a process for the oligomerization of ethylene, said process comprising contacting ethylene with an oligomerization catalyst comprising;
   1) a source of chromium;
   2.1) a first ligand defined by the formula: 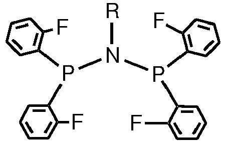 wherein R is isopropyl;
   2.2) a second ligand defined by the formula: 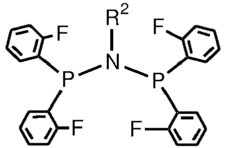 wherein R² is selected from the group consisting of cyclopentyl; a cyclopentyl having at least one C_{1 to 6} alpha substituent; a cyclohexyl and a cyclohexyl using at least one C_{1 to 6} alpha substituent; and
   3) an activator.

### BEST MODE FOR CARRYING OUT THE INVENTION

### PART A CATALYST SYSTEM

The catalyst system used in the process of the present invention must contain three essential components, namely:
(i) a source of chromium;
(ii) two diphosphine ligands; and
(iii) an activator.

Preferred forms of each of these components are discussed below.

### Chromium Source

Any source of chromium that is soluble in the process solvent and which allows the oligomerization process of the present invention to proceed may be used. Preferred chromium sources include chromium trichloride; chromium (III) 2-ethylhexanoate; chromium (III) acetylacetonate and chromium carbonyl complexes such as chromium hexacarbonyl. It is preferred to use very high purity chromium compounds as these should generally be expected to minimize undesirable side reactions. For example, chromium acetylacetonate having a purity of higher than 99% is commercially available (or may be readily produced from 97% purity material - using recrystallization techniques that are well known to those skilled in the art). We have observed that very low Cr concentrations in the reactor are associated with high activity. A range of 0.2 to 8 x 10⁻⁶ molar is suitable, especially from 0.3 to 5.0 x 10⁻⁶.

### Ligands Used in the Oligomerization Process

The first ligand is defined by the formula: 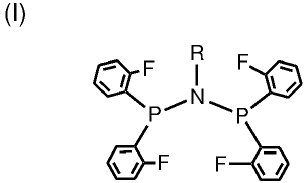 where R is isopropyl.

The second ligand has a bulky hydrocarbyl substituent (R²), on the nitrogen atom and is defined by the formula: 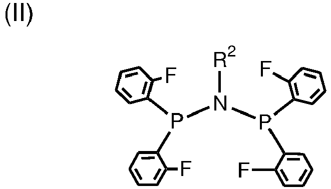 wherein R² is a C₄ to C₂₀ branched hydrocarbyl (such as tertiary butyl) or a cyclic hydrocarbyl. Suitable R² groups are selected from cyclopentyl; a cyclopentyl having at least one C_{1 to 6} alkyl substituent; a cyclohexyl and a cyclohexyl using at least one C_{1 to 6} alkyl substituent.

A ligand in which R² is methylcyclohexyl is suitable and the use of this ligand is shown in the examples.

The first ligand is generally used in an amount of from 90 to 30 mole% with the second ligand being present in an amount of from 10 to 70 %, based on the combined amount of the two ligands. The "total ligand" to chromium ratio is generally from 0.5 to 5/1 (especially from 0.8:1 to 2.0:1) - i.e. where "total ligand" is the combined amount of the first ligand and the second ligand.

### Activator

The activator may be any compound that generates an active catalyst for ethylene oligomerization. Mixtures of activators may also be used. Suitable compounds include organoaluminum compounds and organoboron compounds. Suitable organoaluminium compounds include compounds of the formula AlR₃, where each R is independently C₁-C₁₂ alkyl, oxygen or halide, and compounds such as LiAlH₄ and the like. Examples include trimethylaluminium (TMA), triethylaluminium (TEA), triisobutylaluminium (TIBA), tri-n-octylaluminium, methylaluminium dichloride, ethylaluminium dichloride, dimethylaluminium chloride, diethylaluminium chloride, ethylaluminiumsesquichloride, methylaluminiumsesquichloride, and alumoxanes (also referred to as aluminoxanes). Alumoxanes are well known in the art as typically oligomeric compounds which can be prepared by the controlled addition of water to an alkylaluminium compound, for example trimethylaluminium. Such compounds can be linear, cyclic, cages or mixtures thereof. Commercially available alumoxanes are generally believed to be mixtures of linear and cyclic compounds. The cyclic alumoxanes can be represented by the formula [R⁶AlO]*_{S}* and the linear alumoxanes by the formula R(R⁸AlO)*_{S}* wherein s is a number from about 2 to 50, and wherein R⁶, R⁷, and R⁸ represent hydrocarbyl groups, preferably C₁ to C₆ alkyl groups, for example methyl, ethyl or butyl groups. Alkylalumoxanes especially methylalumoxane (MAO) are preferred.

It will be recognized by those skilled in the art that commercially available alkylalumoxanes may contain a proportion of trialkylaluminium. For instance, some commercial MAO contains approximately 10 wt % trimethylaluminium (TMA), and commercial "modified MAO" (or "MMAO") contains both TMA and TIBA. Quantities of alkylalumoxane are generally quoted herein on a molar basis of aluminium (and include such "free" trialkylaluminium).

A combination of a MAO with additional TEAL is preferred for this invention. The combined use of MAO and TEAL can provide a cost effective cocatalyst system.

In the preparation of the catalyst systems used in the present invention, the quantity of activating compound to be employed is easily determined by simple testing, for example, by the preparation of small test samples which can be used to oligimerize small quantities of ethylene and thus to determine the activity of the produced catalyst. It is generally found that an amount of from 300 to 5000 moles of aluminum per mole of chromium is sufficient. A mix of MAO and TEAL (in which the moles of aluminum that are provided by TEAL are from about 40 to 60 mole % of the total moles of aluminum in the activator) is also suitable. Molar Al/Cr ratios of from 500/1 to 3500/1 are preferred. Additional TEAL increases the total Al/Cr ratio but may actually reduce overall costs as TEAL is much less expensive than MAO.

### PART B CATALYST: RATIOS AND PREPARATION

For comparative oligomerizations at higher temperatures, the chromium and ligand may be present in almost any molar ratio in which the ligand is provided in a molar excess to the chromium. Stated alternatively: a molar equivalent of ligand and chromium provides an active catalyst and excess ligand (though not necessary) does not generally have an adverse impact upon catalyst activity.

A variety of methods are known to purify solvents used to prepare the catalysts including use of molecular sieves (3A), adsorbent alumina and supported de-oxo copper catalyst. Several configurations for the purifier system are known and depend on the nature of the impurities to be removed, the purification efficiency required and the compatibility of the purifier material and the process solvent. In some configurations, the process solvent is first contacted with molecular sieves, followed by adsorbent alumina, then followed by supported de-oxo copper catalyst and finally followed by molecular sieves. In other configurations, the process solvent is first contacted with molecular sieves, followed by adsorbent alumina and finally followed by molecular sieves. In yet another configuration, the process solvent is contacted with adsorbent alumina. One preferred purifier system consists of molecular sieves, followed by adsorbent alumina and finally followed by another set of molecular sieves.

### PART D REACTION CONDITIONS (GENERAL)

Irrespective of the process conditions employed, the oligomerization is typically carried out under conditions that substantially exclude oxygen, water, and other materials that act as catalyst poisons. In addition, the reactor is preferably purged with a nonreactive gas (such as nitrogen or argon) prior to the introduction of catalyst. A purge with a solution of MAO and/or aluminum alkyl may also be employed to lower the initial level of catalyst poisons. Also, oligomerizations can be carried out in the presence of additives to control selectivity, enhance activity and reduce the amount of polymer formed in oligomerization processes.

The process of this invention requires the use of a solvent or diluent because the undesirable formation of C₁₀⁺ oligomers has been observed to increase under continuous flow oligomerization conditions when the concentration of octene in the reactor increases. The addition of a solvent mitigates this problem. Suitable solvents include saturated C₆ to C₂₀ aliphatics (such as hexane, heptane, etc.) and saturated cycloaliphatics (such as cyclohexane or methyl cyclohexane). Unsaturated aliphatics (especially 1-olefins such as 1-hexene; 1-heptene and 1-octene) should be avoided as added solvents/diluents because the use of such unsaturates has been observed to lead to the undesired formation of higher oligomers.

Mixtures of inert diluents or solvents also could be employed. The preferred solvents are aromatic hydrocarbons or saturated aliphatics such as, for example, isobutane, pentane, toluene, xylene, ethylbenzene, cumene, mesitylene, heptane, cyclohexane, methylcyclohexane, chlorobenzene, dichlorobenzene, and mixtures of aliphatics sold under the trademark Isopar®. Cyclohexane and linear C6 to C10 saturated aliphatics are especially preferred. Heptane is an especially preferred linear aliphatic because it is readily separated from the oligomers produced by this reaction using conventional distillation techniques.

The ethylene feedstock for the oligomerization may be substantially pure or may contain other olefinic impurities and/or ethane.

The feedstock is preferably treated to remove catalyst poisons (such as oxygen, water and polar species) using techniques that are well known to those skilled in the art. The technology used to treat feedstocks for polymerizations is suitable for use in the present invention and includes the molecular sieves, alumina and de-oxo catalysts described above for analogous treatment of the process solvent.

### REACTOR

The present invention is typically conducted under batch conditions or continuous flow conditions using a mixed reactor.

Batch reactors are well known. The use of a batch reactor is shown in the Examples.

The term "continuous flow" is meant to convey its conventional meaning - i.e. reactants are continuously added to the reactor and product is continuously withdrawn.

Similarly, the term "mixed reactor" is meant to convey its conventional meaning - i.e. a reactor that contains an agitator or mixing system. A continuously stirred tank reactor ("CSTR") is generally preferred. However, a loop reactor in which mixing is provided by a circulating pump is also suitable (and such reactors are well known to those skilled in the art and are in commercial use).

The use of a CSTR is generally preferred as it is desirable to maintain essentially homogenous reactor conditions - i.e. as will be appreciated by those skilled in the art, a well-mixed CSTR will provide homogenous reactor conditions (in contrast to a plug flow, or tubular reactor, in which the reactor conditions are typically very different at the inlet and discharge). More than one CSTR may be used.

Although a single CSTR is preferred, it is also within the scope of this invention to (optionally) use an additional tubular reactor. If the tubular reactor is employed, it would be placed downstream of the CSTR. The tubular reactor (if used) would provide some additional ethylene conversion, thereby reducing the need to recover/recycle ethylene from the discharge.

### OTHER PROCESS CONDITIONS

A catalyst concentration of from 0.2 to 8 x 10⁻⁶ moles of Cr per litre (micromolar), (especially from 0.3 to 5 micromolar Cr) is suitable.

The reactor temperature is from 20 to 120°C, especially from 35 to 75°C. In general, lower temperatures have been observed to reduce the formation of polymeric byproduct (when other reaction variables are held constant).

Another preferred element of the present invention is the use of ethylene concentrations of 3 to 15 weight %, especially from 5 to 10 weight%. The addition of hydrogen has been observed to reduce the amount of by product polymer that is formed.

The total operating pressure of the process is a function of ethylene concentration, hydrogen concentration and temperature. The use of comparatively low temperature allows a higher ethylene concentration at a given pressure (as ethylene solubility increases at lower temperatures). Preferred operating pressures are from 1 to 20 Mega Pascals (MPa) especially from 2 to 10 MPa.

### PART E REACTOR CONTROL

The control systems required for the operation of agitated reactors are well known to those skilled in the art and do not represent a novel feature of the present invention. In general, temperature, pressure and flow rate readings will provide the basis for most conventional control operations. The increase in process temperature (together with reactor flow rates and the known enthalpy of reaction) may be used to monitor ethylene conversion rates. The amount of catalyst added to the reactor may be increased to increase the ethylene conversion (or conversely, decreased to decrease ethylene conversion) within desired ranges. Thus, basic process control may be derived from simple measurements of temperature, pressure and flow rates using conventional thermocouples, pressure meters and flow meters. Advanced process control (for example, for the purpose of monitoring product selectivity or for the purpose of monitoring process fouling factors) may be undertaken by monitoring additional process parameters with more advanced instrumentation. Known/existing instrumentation that may be employed include in-line/on-line instruments such as NIR infrared, Fourier Transform Infrared (FTIR), Raman, mid-infrared, ultra violet (UV) spectrometry, gas chromatography (GC) analyzer, refractive index, on-line densitometer or viscometer. The use of NIR or GC to measure the composition of the oligomerization reactor and final product composition is especially preferred. A GC analyzer was used to measure the composition of the reactor discharge in the accompanying examples.

The measurement may be used to monitor and control the reaction to achieve the targeted stream properties including but not limited to concentration, viscosity, temperature, pressure, flows, flow ratios, density, chemical composition, phase and phase transition, degree of reaction, polymer content, selectivity.

The control method may include the use of the measurement to calculate a new control set point. The control of the process will include the use of any process control algorithms, which include, but are not limited to the use of PID, neural networks, feedback loop control, forward loop control and adaptive control.

### Catalyst Deactivation, Catalyst Removal and Polymer Removal

In general, the oligomerization catalyst is preferably deactivated immediately downstream of the reactor as the product exits the reaction system. This is to prevent polymer formation and potential build up downstream of the reactor and to prevent isomerisation of the 1-olefin product to the undesired internal olefins. It is generally preferred to flash and recover unreacted ethylene before deactivation. However, the option of deactivating the reactor contents prior to flashing and recovering ethylene is also acceptable. The flashing of ethylene is endothermic and may be used as a cooling source.

In general, many polar compounds (such as water, alcohols and carboxylic acids) will deactivate the catalyst. The use of alcohols, amines and/or carboxylic acids is preferred - and combinations of these are contemplated.

The deactivator may be added to the oligomerization product stream before or after the volatile unreacted reagents/diluents and product components are separated. In the event of a runaway reaction (e.g. rapid temperature rise) the deactivator can be immediately fed to the oligomerization reactor to terminate the reaction. The deactivation system may also include a basic compound (such as sodium hydroxide) to minimize isomerization of the products (as activator conditions may facilitate the isomerization of desirable alpha olefins to undesired internal olefins).

Polymer removal (and, optionally, catalyst removal) preferably follows catalyst deactivation. Two "types" of polymer may exist, namely polymer that is dissolved in the process solvent and non-dissolved polymer that is present as a solid or "slurry".

Solid/non-dissolved polymer may be separated using one or more of the following types of equipment: centrifuge; cyclone (or hydrocyclone), a decanter equipped with a skimmer or a filter. Preferred equipment include so called "self-cleaning filters" sold under the name V-auto strainers, self-cleaning screens such as those sold by Johnson Screens Inc. of New Brighton, Minnesota and centrifuges such as those sold by Alfa Laval Inc. of Richmond, VA (including those sold under the trademark Sharplex®, especially the filters sold with the "auto backwash" feature). The Pall Corporation also sells filters that are suitable for removing solid polymer from the liquid process stream of this invention. The type of filtration technology that is especially suitable for use in the present invention is often referred to as "continuous filtration without filter aid." A specific example of this technology is sold under the trademark Pall ZEF CONTIFLUX®.

Soluble polymer may be separated from the final product by two distinct operations. Firstly, low molecular weight polymer that remains soluble in the heaviest product fraction (C₂₀₊) may be left in that fraction. This fraction will be recovered as "bottoms" from the distillation operations (described below). This solution may be used as a fuel for a power generation system.

An alternative polymer separation comprises polymer precipitation caused by the removal of the solvent from the solution, followed by recovery of the precipitated polymer using a conventional extruder. The technology required for such separation/recovery is well known to those skilled in the art of solution polymerization and is widely disclosed in the literature.

In another embodiment, the residual catalyst is treated with an additive that causes some or all of the catalyst to precipitate. The precipitated catalyst is preferably removed from the product at the same time as by-product polymer is removed (and using the same equipment). Many of the catalyst deactivators listed above will also cause catalyst precipitation. In one embodiment, a solid sorbent (such as clay, silica or alumina) is added to the deactivation operation to facilitate removal of the deactivated catalyst by filtration or centrifugation.

Reactor fouling (caused by deposition of polymer and/or catalyst residue) can, if severe enough, cause the process to be shut down for cleaning. The deposits may be removed by known means, especially the use of high pressure water jets or the use of a hot solvent flush. The use of an aromatic solvent (such as chlorobenzene) for solvent flushing is generally preferred because they are good solvents for polyethylene.

### Product Work Up/Distillation

In one embodiment of the present invention, the oligomerization product produced from this invention is added to a product stream from another alpha olefins manufacturing process for separation into different alpha olefins. As previously discussed, "conventional alpha olefin plants" (wherein the term includes i) those processes which produce alpha olefins by a chain growth process using an aluminum alkyl catalyst, ii) the aforementioned "SHOP" process and iii) the production of olefins from synthesis gas using the so called Lurgi process) have a series of distillation columns to separate the "crude alpha product" (i.e. a mixture of alpha olefins) into alpha olefins (such as butene-1, hexene-1 and octene-1). The mixed hexene-octene product which is preferably produced in accordance with the present invention is highly suitable for addition/mixing with a crude alpha olefin product from an existing alpha olefin plant (or a "cut" or fraction of the product from such a plant) because the mixed hexene-octene product produced in accordance with the present invention can have very low levels of internal olefins. Thus, the hexene-octene product of the present invention can be readily separated in the existing distillation columns of alpha olefin plants (without causing the large burden on the operation of these distillation columns which would otherwise exist if the present hexene-octene product stream contained large quantities of internal olefins). As used herein, the term "liquid product" is meant to refer to the oligomers produced by the process of the present invention which have from 4 to (about) 20 carbon atoms.

In another embodiment, the distillation operation for the oligomerization product is integrated with the distillation system of a solution polymerization plant (as disclosed in Canadian Patent Application No. 2,708,011, Krzywicki et al.).

It will be appreciated that the process solvent must also be separated from the liquid product. This may be done, for example, using distillation. It is highly preferred to recycle the separated solvent back to the oligomerization reactor after it has been distilled/purified.

### EXAMPLES

The following abbreviations are used in the examples:
C = comparative
GC = gas chromatography
Wt = weight
C₄'s = butenes
C₆'s = hexenes
C₈'s = octenes
C10+ = compounds with 10 or more carbons

### Oligomerization Reactions

### EXAMPLES

The first and second ligands are shown below.

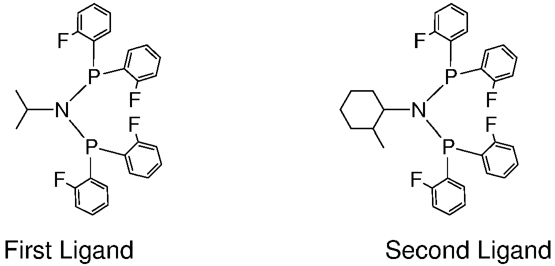

### Part A Ligand Synthesis

The first ligand is a known molecule. The synthesis of this ligand is described, for example, in U.S. 7,994,363 (Carter et al.).

**TABLE 1**

| **Oligomerization Data** | | | | | | | | | |
|---|---|---|---|---|---|---|---|---|---|
| **Run** # | **L1** | **L2** | **Productivity (g/g Cr)** | **PE (g)** | **C4s (wt%)** | **C6s (wt%)** | **C8** | **C-10 & C-10+ (wt%)** | **% 1-C6 in C6 Fraction** |
| 2 | 0.9 | 0.3 | 2,009,788 | 1.0 | 0.10 | 48.19 | 43.16 | 8.43 | 0.99 |
| 3 | 0.6 | 0.6 | 1,896,317 | 1.5 | 0.08 | 53.87 | 37.70 | 8.24 | 0.99 |
| 4 | 0.3 | 0.9 | 1,842,466 | 3.0 | 0.07 | 60.48 | 31.03 | 8.33 | 0.99 |
| 1-C | 1.2 | 0 | 2,102,104 | 0.5 | 0.11 | 42.98 | 47.86 | 8.90 | 0.98 |

*Conditions.* Solvent = cyclohexane, [Cr]=5uM; Ligand/Cr=1.2, MMAO-3A (Al)/Cr=900, Temp. = 60°C, Pressure = 20 bar.

The C₈ fraction was > 99% 1-C₈ in all cases.

### Second Ligand

The second ligand is novel. Synthesis of the second ligand is described below. A schematic of the chemistry to synthesize the second ligand is shown below.

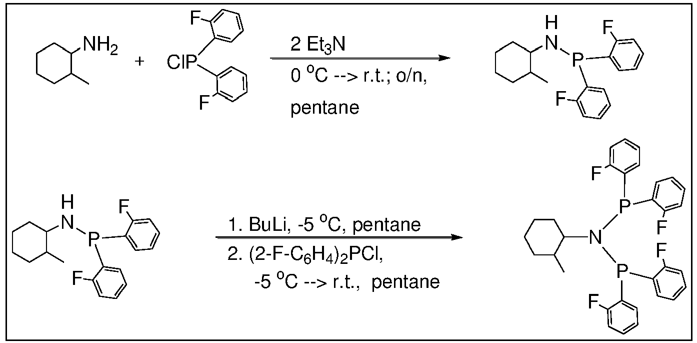

### General Experimental for Ligand Synthesis

All reactions were conducted under nitrogen using standard Schlenk techniques or in an inert atmosphere glovebox. Pentane was purified by placing over activated molecular sieves in an inert atmosphere glovebox. 2-methylcyclohexylamine, triethylamine, and n-butyllithium were purchased from Aldrich and used as is. Deuterated solvents were purchased from Aldrich (dichloromethane-d₂) and were stored over 4 Å molecular sieves. NMR spectra were recorded on a 400 MHz spectrometer (¹H 400.1 MHz).

### 2-Me-Cy-N[P(2-FC₆H₄)₂]₂

To a stirred solution of chlorobis(2-fluorophenyl)phosphine (1.29 g, 5 mmol) and triethylamine (1.02 g [1.5 mL], 10 mmol) in pentane at room temperature was added 10 mL pentane solution of 2-Me-cyclohexylamine (0.571 g, 5 mmol). White precipitate formed part way through the addition of the phosphine. Cooling was removed after addition was completed and the resulting mixture was allowed to warm to room temperature and stirred for 3 hours. The reaction mixture was filtered through a filter cannula. The residue was washed once with 15 mL of fresh pentane. The filtrates (colourless) were combined in a 200-mL Schlenk flask and evaporated to dryness to afford a pale yellow oil. An aliquot was taken for NMR analysis (CC2183-73A). NMR (de121013/1-3, CD₂Cl₂, δ, ppm): ¹H: 7.53 m, 2 H; 7.32 m, 2 H; 7.17 m, 2 H; 6.94 m, 2 H; 2.57 m, 1H; 2.16 br t (J = 10.4 Hz), 1H; 1.65 br m, 4H; 1.26 br m, 4H; 1.03 m, 1H; 0.92 d (J = 6.4 Hz), 1H. ¹⁹F: -107.66 m; -108.1 m. ³¹P{¹H}: 16.82 t (J = 32.4 Hz).

The filtrate from above (CC2183-73A) was cooled (-13 to -5°C) and n-butyllithium (3.2 mL, 1.6 M solution in hexanes, 5 mmol) was added over a 5 minute period. The reaction mixture was kept at this temperature for 45 minutes. A cream colored precipitate formed ∼20 minutes into the experiment. A pentane solution (10 mL) of chlorobis(2-fluorophenyl)phosphine (1.28 g, 5 mmol) was added to this reaction. The precipitate changed to an off-white color. Cooling was removed after the addition was completed and the resulting mixture was allowed to warm to room temperature and stirred overnight. The reaction mixture was filtered through a sintered glass funnel to afford a white residue and colourless filtrate. The filtrate was allowed to slowly concentrate to afford a white solid. (CC2183-73B). NMR (de121021/1-3, CD₂Cl₂, δ, ppm): ¹H: 7.78 brs, 1H; 7.63 brs, 1H; 7.34 m, 5 H; 7.15 m, 3 H; 7.03 m, 2H; 6.94 brs, 4H; 3.15 m, 1H; 2.15, 1H; 1.81 m, 1H; 1.63, m, 3H; 1.50 m, 1H; 1.23 m, 1H; 1.05 m, 1H; 0.90 m, 1H; 0.70 d (J = 6.4 Hz), 3H. ¹⁹F: -102.94 d (J = 206.1 Hz); -104.53 dd (J = 62.8, 261.7 Hz). ³¹P{¹H}: .33.63 s; 22.15 s.

### Part B Ethylene Oligomerization

The present invention provides an oligomerization reaction that produces both octene and hexene. The "alpha purity" of the octene and hexene is high. In many prior art tetramerization processes using P-N-P ligands the alpha purity of the co-produced hexene stream is comparatively low, with (for example) 20 - 35% of the hexene stream consisting of hexene isomers after the hexene-1. For clarity, the term "alpha purity" of a hexene stream represents the weight of hexene-1, divided by the total weight of the hexene stream. Thus, a hexene product that contains 65 weight % hexene-1 and 35 weight% of other hexene isomers is described as having an alpha purity of 65%.

The first ligand ("L₁" in Table 1) and the second ligand ("L₂") were used in several oligomerization experiments. The "total ligand"/chromium ratio was 1.2/1 for all other conditions (for all experiments): chromium concentration was 5 micromolar, Al/Cr = 900 (with a commercially available MAO, sold under the trade name MMAO-3A, used as the source of aluminum in all experiments); temperature = 60°C; pressure = 20 barg; solvent = cyclohexene. As shown in comparative Example 1-C, the alpha purity of the hexene stream (shown as % 1-C₆ in C₆ fractions) falls to 98% under the experimental conditions of this example (whereas alpha purity of greater than 99% has been observed at lower activities). The alpha purity of the hexene stream is increased to 99% for inventive experiments 2- 4. Further details of the experimental conditions follow:
A 600 mL reactor fitted with a stirrer (1750 rpm) was purged 10 times with Argon while at 75°C. The reactor was then cooled to 45°C and purged 3 times with ethylene. The reactor was then cooled to 30°C and depressurized. A solution made up of MMAO-3A (1.278 g of 1.9 wt% Al solution in cyclohexane) and 65 g cyclohexane was transferred via a stainless steel cannula to the reactor. That was followed by an additional 75g of cyclohexane. The reactor was then pressurized with ethylene (approx. 7 barg) and the temperature adjusted to 45°C. A cyclohexane solution (15.8 g) of chromium acetylacetonate (0.349 mg, 0.001 mmol), and diphosphinoamine (PNP) ligand (i-Pr-N[P(2-F-C₆H₄)₂]₂ and [(2-F-C₆H₄)₂P]₂N(2-Me-Cy); different ligand ratios, total ligand/Cr=1.2 mol/mol) was transferred via cannula to a catalyst tower. When the reactor was at 45°C the solution was transferred under ethylene from the catalyst tower to the pressurized reactor. Immediately after, additional ethylene was added to increase the reactor pressure to 20 barg. The reaction was terminated after the reaction had consumed 100 L of ethylene by stopping the flow of ethylene to the reactor and cooling the contents to 30°C, at which point excess ethylene was slowly released from the reactor cooling the contents to 10°C. The product mixture was transferred to a pre-weighed flask. A sample of the liquid product was analyzed by gas chromatography. The solid products were collected, weighed and dried at ambient temperature. The mass of product produced was taken as the difference in weights before and after the reactor contents were added to the flask plus the mass of solid products.

### INDUSTRIAL APPLICABILITY

The linear octene and hexene olefins that are produced by the process of this invention are suitable for a wide variety of end-uses, especially as comonomers for the production of ethylene-alpha olefin copolymers.

## Claims

1. A process for the oligomerization of ethylene, said process comprising contacting ethylene with an oligomerization catalyst comprising;
1) a source of chromium;
2.1) a first ligand defined by the formula: 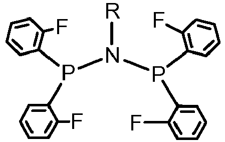 wherein R is isopropyl;
2.2) a second ligand defined by the formula: 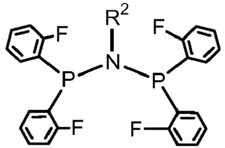 wherein R² is selected from the group consisting of cyclopentyl; a cyclopentyl having at least one C_{1 to 6} alpha substituent; a cyclohexyl and a cyclohexyl using at least one C_{1 to 6} alpha substituent; and
3) an activator

2. The process of claim 1 wherein said activator is methylaluminoxane.

3. The process of claim 1 wherein hydrogen is added.

4. The process of claim 1 wherein said oligomerization conditions comprise a temperature of from 20 to 120°C and a pressure of from 2 to 20 MPa.

5. The process of claim 1 wherein said activator comprises a combination of methylaluminoxane plus triethylaluminium.

6. The process of claim 1, further **characterized in that** the oligomerization rate is greater than 1 million grams of ethylene consumed per hour per gram of chromium.

7. The process of claim 1 wherein R² is selected from the group consisting of 3-methyl cyclohexyl and 2-methyl cyclohexyl.

8. The process of claim 1 wherein the (total moles of ligand 1 plus ligand 2): moles of chromium is from 0.8:1 to 2.0:1.

## Patentansprüche

1. Verfahren zur Oligomerisierung von Ethylen, wobei das Verfahren das Kontaktieren von Ethylen mit einem Oligomerisierungskatalysator umfasst, der Folgendes umfasst:
1) eine Chromquelle;
2.1) einen ersten Liganden, der durch folgende Formel definiert ist: 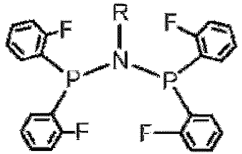 worin R Isopropyl ist;
2.2) einen zweiten Liganden, der durch folgende Formel definiert ist: 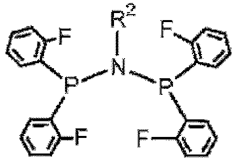 worin R² aus der aus Cyclopentyl, einem Cyclopentyl mit zumindest einem C₁₋₆-α-Substituenten, einem Cyclohexyl und einem Cyclohexyl mit zumindest einem C₁₋₆-α-Substituenten bestehenden Gruppe ausgewählt ist, und
3) einen Aktivator.

2. Verfahren nach Anspruch 1, wobei der Aktivator Methylaluminoxan ist.

3. Verfahren nach Anspruch 1, wobei Wasserstoff zugesetzt wird.

4. Verfahren nach Anspruch 1, wobei die Oligomerisierungsbedingungen eine Temperatur von 20 °C bis 120 °C und einen Druck von 2 bis 20 MPa umfassen.

5. Verfahren nach Anspruch 1, wobei der Aktivator eine Kombination aus Methylaluminoxan und Triethylaluminium umfasst.

6. Verfahren nach Anspruch 1, dass weiters **dadurch gekennzeichnet ist, dass** die Oligomerisierungsrate höher als 1 Million Gramm Ethylen pro Stunde pro Gramm Chrom ist.

7. Verfahren nach Anspruch 1, wobei R² aus der aus 3-Methylcyclohexyl und 2-Methylcyclohexyl bestehenden Gruppe ausgewählt ist.

8. Verfahren nach Anspruch 1, wobei das Verhältnis (mol Ligand 1 + mol Ligand 2 insgesamt) : (mol Chrom) von 0,8:1 bis 2,0:1 reicht.

## Revendications

1. Procédé pour l'oligomérisation d'éthylène, ledit procédé comprenant la mise en contact d'éthylène avec un catalyseur d'oligomérisation comprenant ;
1) une source de chrome ;
2.1) un premier ligand défini par la formule : 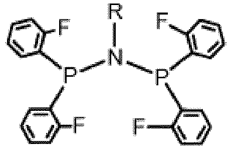 où R est isopropyle ;
2.2) un second ligand défini par la formule : 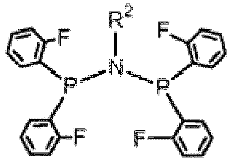 où R² est choisi dans le groupe consistant encyclopentyle ; un cyclopentyle ayant au moins un substituant alpha en C₁ à C₆ ; un cyclohexyle et un cyclohexyle utilisant au moins un substituant alpha en C₁ à C₆ ; et
3) un activateur

2. Procédé selon la revendication 1, dans lequel ledit activateur est le méthylaluminoxane.

3. Procédé selon la revendication 1, dans lequel de l'hydrogène est ajouté.

4. Procédé selon la revendication 1, dans lequel lesdites conditions d'oligomérisation comprennent une température de 20 à 120°C et une pression de 2 à 20 MPa.

5. Procédé selon la revendication 1, dans lequel ledit activateur comprend une combinaison de méthylaluminoxane et de triéthylaluminium.

6. Procédé selon la revendication 1, **caractérisé en outre en ce que** la vitesse d'oligomérisation est supérieure à 1 million de grammes d'éthylène consommés par heure par gramme de chrome.

7. Procédé selon la revendication 1, dans lequel R² est choisi dans le groupe constitué du 3-méthylcyclohexyle et du 2-méthylcyclohexyle.

8. Procédé selon la revendication 1, dans lequel le (nombre total de moles de ligand 1 plus de ligand 2):moles de chrome est de 0,8:1 à 2,0:1.
